(19) Europäisches Patentamt
European Patent Office
Office européen des brevets

(11) EP 1 550 705 B1

(12) EUROPEAN PATENT SPECIFICATION

(45) Date of publication and mention of the grant of the patent:
**09.09.2015 Bulletin 2015/37**

(21) Application number: **03738632.3**

(22) Date of filing: **02.07.2003**

(51) Int Cl.:
***C09K 3/00*** (2006.01) ***A61K 31/715*** (2006.01)
***A61P 37/08*** (2006.01) ***C11D 3/48*** (2006.01)

(86) International application number:
**PCT/JP2003/008390**

(87) International publication number:
**WO 2004/005423 (15.01.2004 Gazette 2004/03)**

# (54) ALLERGEN INACTIVATOR

ALLERGENDESAKTIVATOR

INACTIVATEUR D'ALLERGENES

(84) Designated Contracting States:
**DE FR GB**

(30) Priority: **03.07.2002 JP 2002194588**

(43) Date of publication of application:
**06.07.2005 Bulletin 2005/27**

(73) Proprietor: **KAO CORPORATION**
**Tokyo 103-8210 (JP)**

(72) Inventors:
- **NONOMURA, Mami**
  **HAGA-GUN,**
  **Tochigi 321-3497 (JP)**
- **HORI, Kimihiko**
  **Tokyo 131-8501 (JP)**
- **NOJIRI, Hiroshi**
  **HAGA-GUN,**
  **Tochigi 321-3497 (JP)**

(74) Representative: **Hoffmann Eitle**
**Patent- und Rechtsanwälte PartmbB**
**Arabellastraße 30**
**81925 München (DE)**

(56) References cited:
**WO-A-91/10434**
**WO-A1-00/73351**
**WO-A1-02/28179**
**WO-A1-96/22044**
**JP-A- 58 135 805**
**JP-A- 2001 269 518**
**JP-A- 2002 146 383**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

This invention relates to a polysaccharide derivative use in for inactivating allergens in environment.

### BACKGROUND ART

Recently, allergic diseases such as atopic dermatitis, allergic rhinitis, and asthma are increasing, and such diseases have become an important social problem. One cause for such increase in the allergic diseases is increase of the environmental allergen. In particular, improvement of air tightness of houses has created an ideal condition for mite propagation in the indoor space, and increase in the amount of mite allergen and other allergens in the indoor space has become a serious problem.

Removal of such allergen is a rational means for preventing and treating allergic diseases, and attempts have been made to prevent contact between human and the allergen by using an air cleaner, a highly air tight mattress cover, and the like. The effects, however, have not been sufficient.

Attempts have also been made to inactivate mites by using miticides. Use of such miticides, however, is associated with the risk of adversely affecting human body, and killing of the mites is not the fundamental way of reducing the amount of allergen since feces and corpse remaining after the killing of the mites are allergic.

Attempts have also been made to inactivate the mites by using a repellent. Use of such repellent, however, is associated with the problem of insufficient sustainability of the effects. The number of mites may recover after a while, and even if the number of mites could be reduced, remaining feces and dead body have antigenicity, therefore use of such repellent is not a fundamental way of reducing the amount of the antigen.

Chemical inactivation of the allergen by natural extracts such as tea extract and tannic acid has also been attempted. However, such inactivation is associated with the problem of color development by chemical change with time as well as safety problem when the chemical substance is used in a large amount, therefore use of such chemical substance in a commercial product has been difficult.

EP 1191039 concerns a polysaccharide derivative derived from a polysaccharide or a derivative thereof by substituting a part or all of the hydrogen atoms of the hydroxyl groups of the polysaccharide or the derivative thereof with substituent groups represented by the formula $-E^1-(OA)_n-E^2-R$ wherein $E^1$ represents a linear or branched, divalent, saturated $C_{1-6}$ hydrocarbon group which may be substituted with one or more hydroxyl groups or oxo groups, n represents a number of from 8 to 300, n numbers of As being the same or different and each independently representing a linear or branched, divalent, saturated $C_{1-6}$ hydrocarbon group, $E^2$ represents an ether bond or an oxycarbonyl group (-OCO- or -COO-), and R represents a linear or branched $C_{4-30}$ alkyl group which may be substituted with one or more hydroxyl groups, wherein the hydrogen atom(s) in one or more hydroxy groups in each of the substituent groups (A) may be further substituted with the substituent groups (A) as defined previously. The polysaccharide derivative is employed as a thickener.

WO 91/10434 describes the use of methylcellulose or hydroxyalkyl methylcellulose in the treatment of allergies and other conditions caused by inhalation of airborne irritants.

JP 58 135805 concerns a powdery agent for coating and protecting the nasal mucosa, by using a lower alkyl ether of cellulose such as hydroxypropyl cellulose.

An object of the present invention is to provide a polysaccharide derivative which has no adverse effects on human body and is also free from the problem of the color development for use in inactivating allergens.

### DISCLOSURE OF THE INVENTION

The inventors of the present invention have conducted an extensive search for a substance which is capable of inactivating environmental allergens in a stable manner and which is also highly safe, and found that some particular polysaccharide derivatives have the action of reducing the allergic reaction-inducing capability of the allergen, and that such polysaccharide derivatives are useful as an allergen inactivating agent.

Accordingly, this invention provides a polysaccharide derivative for use in inactivating allergens, wherein said polysaccharide derivative has a cellulose ether or a starch ether as its backbone, and some or all of hydrogen atoms in the hydroxy group of the polysaccharide derivative are substituted by a group represented by the following general formula (1):

$$-E1-(OA)_n-E^2-R \qquad (1)$$

wherein $E^1$ represents an alkylene containing 1 to 6 carbon atoms optionally substituted with hydroxy group or oxo

group; n represents a number of 0 to 50; A independently represents an alkylene containing 1 to 6 carbon atoms, the number of A being n; $E^2$ represents ether bond or oxycarbonyl group; R represents an alkyl group containing 4 to 30 carbon atoms optionally substituted with hydroxy group, a sulfoalkyl group containing 1 to 5 carbon atoms optionally substituted with hydroxy group, or a salt thereof.

This invention also provides embodiments wherein the polysaccharide derivative is contained in a mask or a sheet.

This invention also provides an embodiment wherein the polysaccharide derivative is contained in a cosmetic product.

This invention also provides an embodiment wherein the environment of the allergen is treated with the polysaccharide derivative.

BEST MODE FOR CARRYING OUT THE INVENTION

The polysaccharide derivative of the present invention has excellent water solubility, rheological properties of increase of viscosity at a higher temperature, and excellent emulsifying action, and accordingly, it can be used as a thickener or a stabilizer in various toiletry products including viscous bath agents, cosmetic products used in massaging, shower agents, and skin care agents (WO 00/73351). However, it has been totally unknown that such polysaccharide derivative has the action of reducing the allergic reactions of allergen.

The polysaccharide derivative of the present invention is a polysaccharide derivative having a backbone of cellulose ether or starch ether, and preferable cellulose ether or starch ether is an alkylether wherein a part of the hydrogen atoms on the hydroxy group of the cellulose or the starch have been substituted with an alkyl group and/or a hydroxyalkyl group.

Preferable examples of the cellulose ether include methylcellulose, ethylcellulose, hydroxymethyl cellulose, hydroxyethyl cellulose, hydroxyethyl methylcellulose, hydroxyethyl ethylcellulose, hydroxymethyl hydroxyethyl cellulose, hydroxypropyl cellulose, hydroxypropyl methylcellulose, and the preferred include hydroxyethyl cellulose, hydroxypropyl cellulose, methyl cellulose, and hydroxypropyl methylcellulose.

Preferable examples of the starch ether include methyl starch, ethyl starch, hydroxyethyl starch, hydroxymethyl hydroxyethyl starch, and hydroxypropyl starch, and the preferred includes hydroxyethyl starch and hydroxypropyl starch.

In the cellulose ether or the starch ether as described above, hydroxy group in the hydroxyalkyl group may be further substituted with an alkyl group or a hydroxyalkyl group to form, for example, polyoxyethylene chain.

The degree of substitution by the alkyl group or the hydroxyalkyl group in the cellulose ether or the starch ether of the present invention may exceed 3.0 per constituent monosaccharide residue. The degree of substitution, however, is preferably 0.01 to 3.5, more preferably 0.1 to 3, still more preferably 1 to 3, and even more preferably 1.5 to 2. The weight average molecular weight of the cellulose ether or the starch ether of the present invention is preferably in the range of 10,000 to 2,000,000, more preferably 50,000 to 1,500,000, and even more preferably 100,000 to 600,000.

The polysaccharide derivative of the present invention is the polysaccharide derivative wherein a part or all of the hydrogen atoms in the hydroxy group of the cellulose ether or the starch ether as described above has been replaced with a group represented by the formula (1): $-E^1-(OA)_n-E^2-R$ as shown below, and the degree of substitution is preferably 0.0001 to 1.0, more preferably 0.0005 to 0.5, still more preferably 0.001 to 0.1, and even more preferably 0.001 to 0.05 per constituent monosaccharide residue.

The polysaccharide derivative of the present invention has typical partial structure as shown below when it has hydroxyethyl cellulose for its backbone.

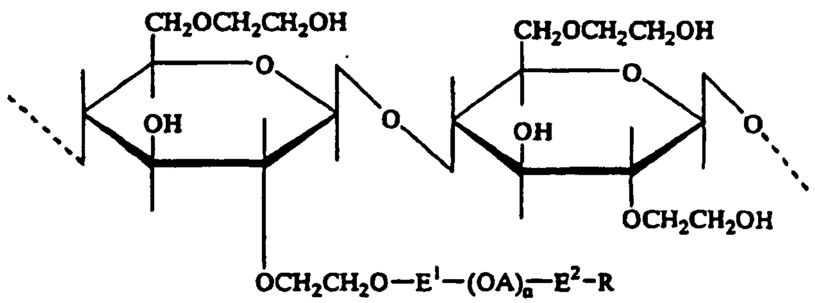

In formula (1), the alkylene group containing 1 to 6 carbon atoms optionally substituted with hydroxy group or oxo group represented by $E^1$ may be either a straight chain or a branched alkylene group, and $E^1$ is preferably a straight chain alkylene group containing 2 to 3 carbon atoms. Exemplary such alkylene groups include ethylene group, propylene

group, trimethylene group, 2-hydroxytrimethylene group, 1-hydroxymethyl ethylene group, 1-oxoethylene group, 1-oxotrimethylene group, and 1-methyl-2-oxoethylene group, and the preferred are 2-hydroxytrimethylene group and 1-hydroxymethyl ethylene group.

In formula (1), the alkylene groups containing 1 to 6 carbon atoms which may be the same or different from each other represented by A may be either a straight chain or a branched alkylene group. A is preferably a straight chain alkylene group containing 2 to 3 carbon atoms. Exemplary such alkylene groups include ethylene group, propylene group, and trimethylene group, and the preferred is ethylene group.

The degree of polymerization of (-OA-) represented by n is 0 to 50. The polymerization degree n, however, is preferably 0 to 40, more preferably 0 to 30, still preferably 0 to 20, still more preferably 10 to 20, and even more preferably 10 to 15 in view of inactivating the allergen. The group A which is included in the number of n may be either the same or different from each other. n is the average addition mole number.

In formula (1), $E^2$ represents ether bond or oxycarbonyl group (-OCO- or -COO-), and $E^2$ is preferably ether bond.

In formula (1), the alkyl group containing 4 to 30 carbon atoms optionally substituted with hydroxy group represented by R may be either a straight chain or a branched alkyl group. R is preferably an alkyl group containing 5 to 25 carbon atoms, more preferably the one containing 6 to 20 carbon atoms, and even more preferably a straight chain alkyl group containing 6 to 20 carbon atoms. Exemplary preferable groups include octyl group, decyl group, dodecyl group, tetradecyl group, hexadecyl group, octadecyl group, and isostearyl group, and dodecyl group, hexadecyl group, and octadecyl group are preferred.

Exemplary sulfoalkyl groups containing 1 to 5 carbon atoms optionally substituted with hydroxy group represented by R include 2-sulfoethyl group, 3-sulfopropyl group, 3-sulfo-2-hydroxypropyl group, and 2-sulfo-1-(hydroxymethyl)ethyl group, and among these, the preferred are 3-sulfo-2-hydroxypropyl group and 2-sulfoethyl group.

The sulfoalkyl group may be partly or entirely in the form of a salt with an element of group I or II such as Na, K, Ca, and Mg, or an organic cation such as an amine or ammonium.

The degree of substitution of the sulfoalkyl group is preferably in the range of 0 to 1.0, more preferably 0 to 0.8, and even more preferably 0 to 0.5 per constituent monosaccharide residue.

The polysaccharide derivative of the present invention can be produced in accordance with the method described in the pamphlet of WO 00/73351. In an exemplary method, the polysaccharide derivative of the present invention is produced by reacting cellulose ether or starch ether with a polyoxyalkylenating agent represented by the following general formula (2):

$$E^3\text{- }(OA)_n\text{-}E^2\text{-}R \qquad (2)$$

wherein $E^3$ is an epoxidated alkyl group containing 3 to 6 carbon atoms; a halogenated alkyl group containing 1 to 6 carbon atoms which is optionally substituted with hydroxy group; or carboxy group, a carboxyalkyl group containing 2 to 6 carbon atoms, or their derivative; n, A, $E^2$, and R are the same as defined above, and if desired, by further reacting with a sulfonating agent (vinylsulfonic acid, a haloalkane sulfonic acid containing 1 to 5 carbon atoms which is optionally substituted with hydroxy group, a sulfonic acid containing 2 to 6 carbon atoms having epoxy group, or their salt).

The polysaccharide derivative as described above has action of reducing or eliminating antigenicity of mite allergens as will be demonstrated in the Examples. Accordingly, the polysaccharide derivative of the present invention is useful as an allergen inactivating agent which reduces or eliminates allergic reaction-inducing capability of various allergens.

The term "allergen" means a substance which causes an allergic reaction such as asthma, allergic rhinitis, pollinosis, or atopic dermatitis upon contact of a human or an animal with such substance. In the present invention, exemplary allergens include plant allergens from plant pollens of Cryptomeria japonica, Chamaecyparis obtusa, ragweed, orchard grass, and the like; animal allergens from epidermis, hair, and parasites of dog, cat, and other animals, insects such as cockroach and moss, mites such as Dermatophagoides, Acarus, and Cryptostigmata; fungi; bacteria; and house dusts (dust, lint, mite feces, and other house dusts).

The term "allergen inactivation" means reducing or elimination of the allergic reaction-inducing capability of the allergen itself, and with regard to animal allergens, such allergen inactivation is clearly different from the action of repellents. More specifically, allergen inactivation can be determined as positive, for example, when allergen is measured by ELISA after treating the mite extract (protein extracted from mite) with 10 folds (weight ratio) of the agent and the quantity (ratio to the control) of Derfl (allergen protein from mite) in relation to the control treated by distilled water is 0.8 or less, more preferably 0.7 or less, and most preferably 0.6 or less. It is to be noted that the expressions such as "covering the allergen", "blocking the allergen", "suppressing the allergen activity ", "converting into a non-allergen", and "reducing the allergen" are equivalent to the allergen inactivation of the present invention.

The allergen inactivating action of the polysaccharide derivative of the present invention is particularly significant on mite allergen, house dust, ceder (Cryptomeria japonica) pollen allergen, and allergens of cat and other pets.

The allergen inactivating agent can be prepared in the form of solution in oil, emulsion, wettable powder, spray,

aerosol, fumigant, coating solution, detergent, powder, or particles by adding emulsifier, fixing agent, dispersing agent, wetting agent, stabilizer, propellant, or the like as desired. More specifically, the allergen inactivating agent can be prepared into household detergent, household softener, detergent for air conditioner filter, household deodorant, reodorant, household bleach, laundry detergent, softener, laundry starch, laundry deodorant, laundry bleach, paper products for house cleaning, kitchen detergent, kitchen bleach, mask spray, or the like, and the desired effect can be obtained by applying the product in various environment where allergen is present, for example, by distributing, spraying, coating, evaporating such product on floor, tatami mat, carpet, mattress, rug, tatami mat, wall, bed, sofa, pillow, or in closet, by washing clothing or curtain with the product, by treating filter in the air cleaner, fabric such as mattress cover, sheets, or pillow, materials such gauze or nonwoven cloth used in the mask with the product.

Addition of a repellent, an insecticide, or other agents for mite, moss, cockroach, and other arthropods to the preparation as described above in addition to the polysaccharide derivative of the present invention is effective, and examples of such agents include insecticide, repellent, synergist, bactericide, fungicide, activating agent, deodorant, and flavoring agent for mite, moss, cockroach.

Exemplary miticides include synthetic pyrethroid such as d-phenothrin (3-phenoxybenzyl d-cis/trans-chrysanthemate), permethrin (3-phenoxybenzyl dl-cis/trans-2,2-dimethyl-3-(2',2'-dichlorovinyl)-cyclopropane carboxylate), resmethrin ((5-benzyl-3-furil) methyl dl-cis/ trans-chrysanthemate), allethrin (dl-3-allyl-2-methyl-4-oxo-2-cyclopentenyl dl-cis/trans-chrysanthemate), phthalthrin ((N-3,4,5,6,-tetrahydro-phthalimide) methyl dl-cis/trans-chrysanthemate), empenthrin (1-ethinyl-2-methyl-2-pentenyl dl-cis/trans-chrysanthemate), and d,dT80-pralethrin (d-2-methyl-4-oxo-3-propargylcyclopent-2-enyl d-cis/trans-chrysanthemate) and their derivatives, and antimite reagents derived from a natural essential oil such as hinokitiol, benzyl benzoate, and jasmonic acid derivative.

Exemplary mite repellents that may be used include diethylamide, dimethyl phthalate, dibutylphthalate, MGK repellent 326, dubtlex, 2-ethyl-1,3-hexanediol.

Exemplary miticide synergist and/or miticide include piperonyl butoxide, octachlorodipropylether, N-(2-ethylhexyl)-1-isopropyl-4-methylbicyclo [2,2,2] octo-5-en-2,3-dicarboxyimide, and N-(2-ethinyl)-bicyclo [2,2,1]-hepta-5-en-2,3-dicarboxyimide.

Examples of bactericide and fungicide which suppress proliferation of the fungi or the bacteria having antigenicity by itself and being fed on by the house dust mites include tiabendazole, triclosan, chlorhexidine, zinc pyrithione, chloroxylenol, densil, benzalkonium chloride, dichlofluanid, sodium benzoate, p-methyl oxybenzoate, phenoxy ethanol, and ethanol, as well as natural components such as chitosan, catechin, thymol, hinokitiol, Phyllostachys extract, mustard essential oil, and wasabi essential oil.

The preparation as described above may contain the polysaccharide derivative of the present invention in combination with a known anti-allergen reagent such as tannic acid, tea extract, hydroxyapatite, epicatechin, epigallocatechin gallate, epigallocatechin gallate, or gallic acid (Japanese Patent Application Laid-Open No. 6-279273); smectite or other clay mineral which is an allergen capturing reagent; or a hydroxybenzoate compound which is known to be an allergen removing agent (Japanese Patent Application Laid-Open No. 11-292714).

A mask and other products having the allergy preventing effect may be produced by spraying or impregnating the allergen inactivating agent to a mask or a sheet used therefor such as gauze or nonwoven. The preparation used for the spraying or impregnation is preferably a mixture based on water or alcohol, and the alcohol is preferably ethanol, propanol, isopropanol, or 1,3-butylene glycol.

The sheet material used for constituting the mask (sheet for the mask) may be any material as long as it is air permeable, and the sheet material may be, for example, a woven fabric such as gauze, a nonwoven, or a paper (such as pulp paper or rayon fiber paper). Preferable materials include dry nonwovens such as thermally bonded nonwoven, spunlace nonwoven, and chemical bond nonwoven and wet nonwovens such as spunbond nonwoven, and meltblown nonwoven. The fiber constituting the nonwoven may be a thermoplastic fiber such as polyester fiber, polyamide fiber, or polyolefin fiber; their complex fiber or split fiber; a semi-synthetic fiber such as acetate; a regenerated fiber such as cupura or rayon; a natural fiber such as cotton or pulp; a mixture thereof, and the type of the fiber used may be adequately selected depending on the production method.

Such sheet for the mask may be fabricated into the mask product by attaching ear hangers to the sheet, by forming ear hanger holes in the sheet material itself, or by inserting the sheet as an auxiliary sheet between the mask and the mouth (on the surface or in the interior of the mask or at the part of the mask that corresponds to the mouth).

In such a case, the polysaccharide derivative is preferably incorporated in the allergen inactivating agent at an amount of 0.001 to 30% by weight, and preferably at 0.01 to 5% by weight, and the amount of the allergen inactivating agent impregnated in the sheet is preferably 0.01 to 60 folds, and more preferably 0.1 to 10 folds of the sheet weight.

Conventional masks for pollinosis are associated with the risk that symptoms of allergic diseases may be induced when the pollen, mite, or other allergen that has been captured by the mask is released from the mask and inhaled by the patient. In contrast, the mask and the sheet for the mask of the present invention have the merit that such allergic symptoms are less likely to be induced even if the allergen that had been captured was released from the mask since the captured allergen is detoxidated as soon as it is captured by the mask.

Alternatively, the allergen inactivating agent may be directly applied to the skin as an external agent as in the case of cosmetic product, for example, in the form of water-in-oil or oil-in-water cosmetic emulsion, cream, gel, cosmetic emulsion, lotion, oily cosmetic product, facial wash, foundation, pack, cataplasm, spray, mist, lip stick, hairtonic, hairdressing, shampoo, hair rinse, hair conditioner, and other skin detergent.

Such cosmetic product may be formulated by combining the allergen inactivating agent with any desired components commonly used in the cosmetic products such as oil content, ceramide, pseudoceramide, sterol, moistening agent, antioxidant, singlet enzyme quencher, powder component, colorant, UV absorber, whitening agent, alcohols, chelating agent, pH adjusting agent, preservative, thickener, pigment, flavor, plant extract, various skin nutrients.

Amount of the polysaccharide derivative of the present invention incorporated in the preparation as described above may be adequately determined depending on the dosage form, the treatment method, and the site of the treatment. The polysaccharide derivative, however, is preferably incorporated at 0.001 to 20% by weight, and more preferably at 0.01 to 10% by weight of the entire composition. When the preparation is used without dilution, the amount incorporated is preferably in the range of 0.01 to 2% by weight, and when the diluted preparation is used, the polysaccharide derivative is preferably incorporated so that the amount is 0.1 to 10% by weight in the stock solution, and the stock solution is preferably diluted to 10 to 10,000 fold before its use.

When the allergen inactivating agent is used for external agent applied on the skin as in the case of cosmetic product, the allergen inactivating agent is preferably incorporated in the product at an amount of 0.001 to 20% by weight, and more preferably at 0.01 to 10% by weight.

EXAMPLES

Next, the present invention is described in further detail by referring to the Examples.

Production Example 1

80 g of hydroxyethyl cellulose (HEC-QP100MH manufactured by Union Carbide Company) having a weight average molecular weight of 1,500,000 and degree of substitution by hydroxyethyl group of 1.8, 640 g of 80% isopropyl alcohol, and 5.34 g of 48% aqueous solution of sodium hydroxide were mixed to produce a slurry, and the slurry was stirred in nitrogen atmosphere at room temperature for 30 minutes. To this solution was added 12.78 g of polyoxyalkylenating agent represented by the following formula:

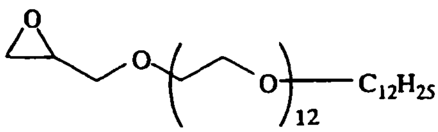

and the mixture was allowed to react at 80°C for 8 hours for polyoxyalkylenation. After the termination of the reaction, the reaction solution was neutralized with acetic acid, and the reaction product was separated by filtration. The reaction product was washed twice with 500 g of isopropyl alcohol, and dried under reduced pressure at 60°C for one day to produce 72.0 g of polyoxyalkylenated hydroxyethyl cellulose derivative (Compound 1).

The degree of substitution of the substituent containing the polyoxyalkylene group in the resulting hydroxyethyl cellulose derivative was 0.004.

Production Example 2

Compounds 2 to 16 shown in Table 1 were produced by the methods described in Production Example 1 and the pamphlet of WO 00/73351.

Production Example 3

(1) To a 1000 mL glass separable reaction vessel equipped with agitator, thermometer, and condenser were added 80 g of hydroxyethyl cellulose (HEC-QP100M manufactured by Union Carbide Company) having a weight average molecular weight of about 1,500,000 and degree of substitution by hydroxyethyl group of 1.8, 640 g of 80% isopropyl alcohol, and 5.5 g of 48% aqueous solution of sodium hydroxide to prepare a slurry, and the slurry was stirred in nitrogen atmosphere at room temperature for 30 minutes. To this solution was added 2.52 g of stearyl glycidylether,

and the mixture was allowed to react at 80°C for 8 hours for hydrophobicization. After the termination of the hydrophobicization reaction, the reaction solution was neutralized with acetic acid, and the reaction product was separated by filtration. The reaction product was washed twice with 500 g of isopropyl alcohol at 50°C, and then, twice with 500 g of acetone, and dried under reduced pressure at 70°C for one day to produce 72.8 g of hydrophobicized hydroxyethyl cellulose derivative.

(2) To a 500 mL glass separable reaction vessel equipped with agitator, thermometer, and condenser were added 20.0 g of hydrophobicized hydroxyethyl cellulose derivative produced in (1), 200 g of 70% isopropyl alcohol, and 1.37 g of 48% aqueous solution of sodium hydroxide to produce a slurry, and the slurry was stirred in nitrogen stream at room temperature for 30 minutes. To the reaction solution were added 28 g of sodium 3-chloro-2-hydroxypropane sulfonate and 11.9 g of 48% aqueous solution of sodium hydroxide, and sulfonation was allowed to proceed at 50°C for 3 hours. After the termination of the reaction, the reaction solution was neutralized with hydrochloric acid and the reaction product was separated by filtration. The reaction product was washed once with 340 g of 70% isopropyl alcohol, and then twice with 120 g of isopropyl alcohol, dried under reduced pressure at 70°C for 1 day to produce 18.3 g of hydroxyethyl cellulose derivative substituted with 3-stearyloxy-2-hydroxypropyl group and 3-sulfo-2-hydroxypropyl group (Compound 17) .

The resulting hydroxyethyl cellulose derivative had a degree of substitution by 3-stearyloxy-2-hydroxypropyl group of 0.003, and a degree of substitution by 3-sulfo-2-hydroxypropyl group of 0.210.

Production Example 4

Compound 18 shown in Table 1 was produced by the method of Production Example 3.

Production Example 5

80 g of hydroxyethyl cellulose (HEC-QP15000H manufactured by Union Carbide Company) having a weight average molecular weight of about 800,000 and degree of substitution by hydroxyethyl group of 1.8, 640 g of 80% isopropyl alcohol, and 2.0 g of p-toluenesulfonic acid were mixed to produce a slurry, and the slurry was stirred in nitrogen atmosphere at room temperature for 30 minutes. To this solution was added 15 g of the compound represented by the following formula:

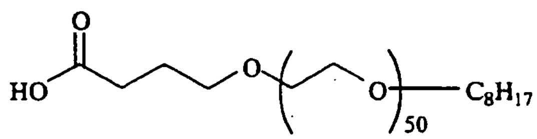

and the mixture was allowed to react at 80°C for 8 hours for polyoxyalkylenation. After the termination of the reaction, the reaction solution was neutralized with 48% aqueous solution of sodium hydroxide, and the reaction product was separated by filtration. The reaction product was washed twice with 500 g of isopropyl alcohol, then twice with 500 g of isopropyl alcohol, and dried under reduced pressure at 70°C for one day to produce 73.4 g of hydroxyethyl cellulose derivative (Compound 19).

The degree of substitution of the substituent containing the polyoxyalkylene group in the resulting hydroxyethyl cellulose derivative was 0.010.

Production Example 6

(1) 80 g of potato starch (manufactured by Katayama Chemical, Inc.), 640 g of 50% isopropyl alcohol, and 5.5 g of 48% aqueous solution of sodium hydroxide were mixed to produce a slurry, and the slurry was stirred in nitrogen atmosphere at room temperature for 30 minutes. To this solution was added 19.0 g of polyoxyalkylenating agent represented by the following formula:

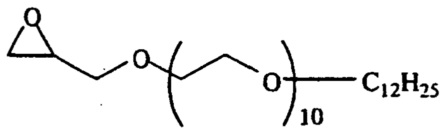

and the mixture was allowed to react at 80°C for 8 hours for polyoxyalkylenation. After the termination of the reaction, the reaction solution was neutralized with acetic acid, and the reaction product was separated by filtration. The reaction product was washed twice with 500 g of 50% isopropyl alcohol, and then twice with 500g of acetone, and dried under reduced pressure at 70°C for one day to produce 69.4 g of starch derivative.
The degree of substitution of the substituent containing the polyoxyalkylene group in the resulting starch derivative was 0.005.
(2) 35.5 g of the polyoxyalkylenated starch produced in (1), 350 g of 70% isopropyl alcohol, and 2.4 g of 48% aqueous solution of sodium hydroxide were mixed to produce a slurry, and the slurry was stirred in nitrogen atmosphere at room temperature for 30 minutes. To the reaction solution were added 25.1 g of sodium monochloroacetate and 18.0 g of 48% aqueous solution of sodium hydroxide, and carboxymethylation was allowed to take place at 50°C for 5 hours. After the termination of the reaction, the reaction solution was neutralized with acetic acid, and the resulting product was separated by filtration. The resulting product was washed three times with 400 g of 70% isopropyl alcohol, then twice with 300 g of isopropyl alcohol, and dried under reduced pressure at 70°C for 1 day to produce 33.8 g of starch derivative which had been polyoxyalkylenated and carboxymethylated (Compound 20). The resulting starch derivative had degree of carboxymethylation of 0.48.

Table 1

| Compound | Code number | Backbone (Cellulose ether or starch ether) | | | Substituent $[-E^1-(OA)_nE^2-R-]$ | | | | | |
|---|---|---|---|---|---|---|---|---|---|---|
| | | Substance | Average molecular weight | Degree of alkyl substitution | $E^1$ | A | n | $E^2$ | R | Degree of substitution |
| Compound 1 | EPS-11 | Hydroxyethyl cellulose (UCC) | 1,500,000 | 1.8 | 2-hydroxy-trimethylene | Ethylene | 12 | -O- | $-nC_{12}H_{25}$ | 0.004 |
| Compound 2 | EPS-21 | Hydroxyethyl cellulose (UCC) | 500, 000 | 1.8 | 2-hydroxy-trimethylene | Ethylene | 12 | -O- | $-nC_{12}H_{25}$ | 0.004 |
| Compound 3 | EPS-49 | Hydroxyethyl cellulose (Hercules) | 500,000 | 2.5 | 2-hydroxy-trimethylene | Ethylene | 12 | -O- | $-nC_{12}H_{25}$ | 0.004 |
| Compound 4 | EPS-47 | Hydroxyethyl cellulose (Hercules) | 200,000 | 2.5 | 2-hydroxy-trimethylene | Ethylene | 12 | -O- | $-nC_{12}H_{25}$ | 0.014 |
| Compound 5 | EPS-63 | Hydroxyethyl cellulose (Hercules) | 100,000 | 2.5 | 2-hydroxytrime thylene | Ethylene | 12 | -O- | $-nC_{12}H_{25}$ | 0.02 |
| Compound 6 | EPS-28 | Hydroxypropyl starch (Nippon Starch Chemical Co., Ltd.) | Unknown | Unknown | 2-hydroxy-trimethylene | Ethylene | 12 | -O- | $-nC_{12}H_{25}$ | 0.007 |
| Compound 7 | - | Hydroxyethyl cellulose (UCC) | 800,000 | 1.8 | 2-hydroxy-trimethylene | Ethylene | 20 | -O- | $-nC_{18}H_{37}$ | 0.003 |
| Compound 8 | EPS-33 | Methyl cellulose (Shin-Etsu Chemical Co., Ltd.) | 1000, 000 | Mel.4 Hydroxy-propyl 0.2 | 2-hydroxy-trimethylene | Ethylene | 12 | -O- | $-nC_{12}H_{25}$ | 0.004 |
| Compound 9 | EPS-1 | Hydroxyethyl cellulose (UCC) | 1500, 000 | 1.8 | 2-hydroxy-trimethylene | Ethylene | 9 | -O- | $-nC_{12}H_{25}$ | 0.0055 |
| Compound 10 | EPS-31 | Hydroxyethyl cellulose (UCC) | 800,000 | 1.8 | 2-hydroxy-trimethylene | Ethylene | 12 | -O- | $-nC_{12}H_{25}$ | 0.0041 |
| Compound 11 | EPS-35 | Hydroxyethyl cellulose (UCC) | 1500,000 | 1.8 | 2-hydroxy-trimethylene | Ethylene | 19 | -O- | $-nC_{12}H_{25}$ | 0.004 |
| Compound 12 | EPS-42 | Hydroxyethyl cellulose (Hercules) | 100.000 | 2.5 | 2-hydroxy-trimethylene | Ethylene | 12 | -O- | $-nC_{12}H_{25}$ | 0.004 |

(continued)

| Compound | Code number | Backbone (Cellulose ether or starch ether) | | | Substituent $[-E^1-(OA)_nE^2-R-]$ | | | | | |
|---|---|---|---|---|---|---|---|---|---|---|
| | | Substance | Average molecular weight | Degree of alkyl substitution | $E^1$ | A | n | $E^2$ | R | Degree of substitution |
| Compound 13 | EPS-44 | Hydroxyethyl cellulose (UCC) | 500.000 | 1.8 | 2-hydroxy-trimethylene | Ethylene | 15 | -O- | $-nC_{16}H_{33}$ | Unknown |
| Compound 14 | EPS-62 | Hydroxyethyl cellulose (Hercules) | 100.000 | 2.5 | 2-hydroxy-trimethylene | Ethylene | 12 | -O- | $-nC_{12}H_{25}$ | 0.0123 |
| Compound 15 | EPS-41 | Hydroxyethyl cellulose (Hercules) | 200.000 | 2.5 | 2-hydroxy-trimethylene | Ethylene | 12 | -O- | $nC_{12}H_{25}$ | 0.004 |
| Compound 16 | EPS-46 | Hydroxyethyl cellulose (Hercules) | 200.000 | 2.5 | 2-hydroxy-trimethylene | Ethylene | 12 | -O- | $-nC_{12}H_{25}$ | 0.009 |
| Compound 17 | SPS-K1 | Hydroxyethyl cellulose (UCC) | 1.500.000 | 1.8 | 2-hydroxy-trimethylene | - | 0 | -O- | $-nC_{18}H_{37}$ | 0.0032 |
| Compound 18 | SPS-S | Hydroxyethyl cellulose (Hercules) | 1.500.000 | 2.5 | 2-hydroxy-trimethylene | - | 0 | -O- | $-nC_{18}H_{37}$ | 0.0037 |
| Compound 19 | - | Hydroxyethyl cellulose (UCC) | 800.000 | 1.8 | $-(CH_2)_3CO-$ | Ethylene | 50 | -O- | $-nC_8H_{17}$ | 0.010 |
| Compound 20 | - | Potato starch (Katayama Chemical, Inc.) | - | - | 2-hydroxy-trimethylene | Ethylene | 10 | -O- | $-nC_{12}H_{25}$ | 0.005 |
| Compound 21*1 | POLY SURF67 | Hydroxyethyl cellulose | Unknown | Unknown | Unknown | Unknown | 0 | Unknown | Unknown | Unknown |
| Compound 22*2 | PLUS 330CS | Hydroxyethyl cellulose | Unknown | Unknown | Unknown | Unknown | 0 | Unknown | Unknown | Unknown |

*1: POLY SURF 67 (manufactured by Hercules Incorporated), *2: PLUS 330CS (manufactured by Hercules Incorporated)

Example 1

(1) The polysaccharide derivative of the present invention was dissolved in distilled water to prepare 1% solution. Quantity of Dermatophagoides allergen was measured as a typical allergen in the indoor environment by sandwich ELISA using a mouse monoclonal antibody. More specifically, quantity of Dermatophagoides allergen Derf2 was measured by using Derf2 antibody and a labeled antibody manufactured by Asahi Brewery, Ltd.
The allergen inactivation effect is indicated in terms of the ratio in relation to the control wherein the sample was treated in the same way except for the use of distilled water, namely, by assuming the Derf2 quantity in the control as 1.
(2) Scratch extract "mite" (manufactured by Torii Pharmaceutical Co., Ltd.) was placed in a dialysis tube, and dialyzed overnight in 10% PBS (4°C) to remove glycerol in the extract. The thus dialyzed mite extract was adjusted to a concentration of 0.5 mg/mL with PBS. 50 μL of this mite extract and 50 μL of 1% sample solution prepared with distilled water were placed in a 1. 5 mL siliconized microtube, and the mixture was stirred with vortex and allowed to stand at room temperature for 2 hours. For the control, the sample was replaced with distilled water of the same volume. 1% tannic acid of the same volume was used for the positive control. The reaction was then terminated by adding 400 μL of 11.25% BSA (dissolved in PBS) to the tube, and centrifugation was conducted at 15,000 rpm at room temperature for 10 minutes. The supernatant was used for ELISA. Quantity of Derf2 in the reaction solution was determined by using anti-Derf2 monoclonal antibody (15E11) and HRP-labeled anti-Derf2 monoclonal antibody (13A4) manufactured by Asahi Brewery, Ltd. in accordance with the protocol attached with the product, and using Derf2 for the Derf2 antigen for depicting the calibration curve.

Quantity of the Derf2 treated with various samples was calculated in relation to the quantity of Derf2 treated with the distilled water, which was assumed to be 1. The results are shown in Table 2.

Table 2

| Sample | Quantity of Derf2 (Ratio to the control) |
|---|---|
| Compound of the invention | |
| Compound 9 | 0.6 |
| Compound 1 | 0.6 |
| Compound 6 | 0.4 |
| Compound 10 | 0.6 |
| Compound 11 | 0.5 |
| Compound 12 | 0.6 |
| Compound 13 | 0.6 |
| Compound 21 | 0.8 |
| Compound 22 | 0.8 |
| Comparative compound | |
| Tannic acid | 0.2 |
| Distilled water | 1.0 |

As described above, the compounds of the present invention were highly effective in inactivating the allergen.

Example 2

Quidel Allergen Screen (manufactured by Xenith Biomed) which is a reagent for detecting antigen-specific IgE antibody by using enzyme-labeled anti-IgE antibody was used to measure the reactivity between the allergen (house dust, Dermatophagoides farinae, Dermatophagoides pteronyssinus, cat epidermis, Cryptomeria japonica, ragweed, and the like) immobilized on a dip stick and the IgE antibody from the allergy patient by the procedure as described below.
The allergen stick was placed in a wetting box with the pad side facing upward, and the stick was impregnated with the product of the present invention adjusted to 1% at an amount of 100 μL/stick and left at room temperature for

2 hours. Each pad was washed evenly for 30 seconds with physiological saline in a washing bottle, and 50 μL/stick of the serum collected from an allergy patient was added dropwise to the pad and the serum was evenly spread. The wetting box was covered with lid and allowed to stand for 18 hours at room temperature. After the completion of the reaction, the pad was washed evenly for 20 seconds by the physiological saline in a washing bottle.

About 1 mL of enzyme-labeled anti-IgE antibody was placed in a test tube, and allergen stick is placed in the test tube with the pad facing downward after washing the allergen stick and shaking off the excess water, and the reaction was allowed to take place at room temperature for 30 minutes. After the termination of the reaction, each pad was evenly washed with tap water for 2 minutes. At this occasion, disappearance of the red color on the pad was confirmed. About 1 mL of the substrate solution was poured into test tube, and allergen stick was placed in the test tube with the pad facing downward after washing the allergen stick and shaking off the excess water, and the reaction was allowed to take place at room temperature for 30 minutes. After the completion of the reaction, reaction was terminated by absorbing the water impregnated in the pad with a paper towel by gently patting the pad with the paper towel from the rear side of the pad. Next, intensity of the blue color developed was measured with an image analyzer, and decrease in the reactivity by the product of the present invention was calculated by the following equation by using the color intensity attained when the IgE from allergy patient was used with distilled water treatment as the control. The results are shown in Tables 3 and 4.

Allergen inactivation effect (%) =

100 - {(IgE reaction intensity when treated by the product of the present invention) - (reaction intensity of the negative control)}/{(IgE reaction intensity of the control treated by distilled water) -(reaction intensity of the negative control) x 100

Table 3

| Allergen inactivating effect (%) <Animal allergen> | | | | |
|---|---|---|---|---|
| Sample | House dust 1 | Dermatophagoides farinae | Dermatophagoides pteronyssinus | Cat epithelium |
| Compound of the invention | | | | |
| Compound 1 | 86 | 80 | 90 | 87 |
| Compound 4 | 100 | 100 | 100 | 99 |
| Compound 17 | 52 | 46 | 51 | 48 |
| Compound 18 | 97 | 98 | 98 | 96 |
| Compound 14 | 99 | 98 | 97 | 98 |
| Compound 2 | 99 | 95 | 95 | 97 |
| Compound 15 | 97 | 89 | 85 | 97 |
| Compound 16 | 99 | 81 | 82 | 97 |
| Compound 3 | 100 | 97 | 96 | 100 |
| Compound 21 | 92 | 87 | 86 | 98 |
| Compound 22 | 62 | 62 | 60 | 77 |
| Comparative compound | | | | |
| Tannic acid | 83 | 62 | 70 | 92 |
| Smectite | 40 | 31 | 55 | 23 |

(continued)

| | | | | |
|---|---|---|---|---|
| Comparative compound | | | | |
| Distilled water | 0 | 0 | 0 | 0 |

Table 4

| Allergen inactivating effect (%) <Plant allergen> | | |
|---|---|---|
| Sample | Ceder | Ragweed |
| Compound of the invention | | |
| Compound 1 | 70 | 99 |
| Compound 4 | 90 | 100 |
| Comparative compound | | |
| Tannic acid | 78 | Nd |
| Smectite | 50 | 75 |
| Distilled water | 0 | 0 |

As described above, the compound of the present invention had high allergen reducing effect.

Example 3: Production of mask for pollinosis

Materials used in commercially available masks such as gauze and nonwoven are impregnated with Preparation 1 as shown below containing the compound of the present invention (Compound 1, Compound 3, Compound 4, Compound 14, or Compound 21) at an amount approximately 3 times larger than the material, and the impregnated materials are dried or semi-dried to produce the mask for pollinosis. Use of such mask is effective in capturing and detoxicating the pollen.

<Preparation 1>

| | |
|---|---|
| The compound of the present invention | 1% |
| 1, 3-butylene glycol | 20% |
| Methyl paraoxybenzoate | 0.2% |
| Piroctone olamine | 0.1% |
| Sodium benzoate | 0.2% |
| Lysine | 1.0% |
| Flavor | 0.1% |
| Purified water | the balance |

Example 4: Production of sheet for pollinosis mask

A sheet for pollinosis mask can be produced by impregnating a sheet of nonwoven used for the mask with Preparation 1 as described above of the amount approximately 3 times the mass of the sheet, and drying or semi-drying the sheet. Use of such sheet by sandwiching the sheet between the mask or by covering the mouth with the sheet is effective in capturing and detoxicating the pollen.

Example 5: Spraying solution for mask

Preparation 2 as shown below containing the compound of the present invention (Compound 1, Compound 3, Compound 4, Compound 14 or Compound 21) is produced to use the preparation as a solution for spraying the mask.

By spraying of this spraying solution on a commercially available mask and using the mask after drying, effects equivalent to those of the pollinosis mask and the sheet for pollinosis mask as described above can be expected.

<Preparation 2>

| | |
|---|---|
| The compound of the present invention | 0.5% |
| 55% ethanol | 50% |
| Glycerin | 2% |
| Flavor | 0.1% |
| Purified water | the balance |

Example 6: Cosmetic product

Cosmetic products containing the compounds of the present invention (Compound 1, Compound 3, Compound 4, Compound 14, Compound 17, Compound 18 or Compound 21) and having the formulation as shown in (1) and (2), below are produced by the method commonly used in the art. By applying these cosmetic products on the skin, mite allergen which is the cause of atopic dermatitis can be detoxicated on the skin to thereby prevent the onset of dermatitis or improve the dermatitis.

(1) Protective cream

| | |
|---|---|
| The compound of the present invention | 1 g |
| Cholesterol | 0.5 g |
| Cholestearyl isostearate | 1 g |
| Polyether modified silicone | 1.5 g |
| Cyclic silicone | 20 g |
| Methylphenyl polysiloxane | 2 g |
| Methyl polysiloxane | 2 g |
| Magnesium sulfate | 0.5 g |
| 55% ethanol | 5 g |
| Carboxymethyl chitin | 0.5 g |
| Ceramide | 0.5 g |
| Purified water | balance/total 100 g |

(2) Protective lotion

| | |
|---|---|
| The compound of the present invention | 2 g |
| Sodium pyrroridonnecarboxylate | 1 g |
| Polyoxyethylene (20) sorbitan monolaurate | 1.5 g |
| Glycerin | 2 g |
| Purified water | balance/total 100 g |

INDUSTRIAL APPLICABILITY

Use of the polysaccharide derivative of the present invention enables inactivation of house dust and other allergens in the environment without causing adverse effects on human body or problems such as color development.

## Claims

1. A polysaccharide derivative having a cellulose ether or a starch ether as its backbone, wherein some or all of hydrogen atoms in the hydroxy group of the polysaccharide derivative are substituted by a group represented by the following general formula (1):

$$-E^1-(OA)_n-E^2-R \qquad (1)$$

wherein $E^1$ represents an alkylene containing 1 to 6 carbon atoms optionally substituted with hydroxy group or oxo group; n represents a number of 0 to 50; A independently represents an alkylene containing 1 to 6 carbon atoms, the number of A being n; $E^2$ represents an ether bond or an oxycarbonyl group; R represents an alkyl group containing 4 to 30 carbon atoms optionally substituted with hydroxy group, a sulfoalkyl group containing 1 to 5 carbon atoms optionally substituted with hydroxy group, or a salt thereof for use in inactivating allergens.

2. The polysaccharide derivative for use according to claim 1, **characterized in that** the allergen to be inactivated is selected from plant allergen, animal allergen, fungi, bacterium, and house dust.

3. The polysaccharide derivative for use according to claim 1 or claim 2, **characterized in that** said cellulose ether or starch ether is hydroxyethyl cellulose.

4. The polysaccharide derivative for use according to any one of claims 1 to 3, **characterized in that** the inactivation of allergens is the detoxication of mite or pollen allergen.

5. The polysaccharide derivative for use according to any one of claims 1 to 3, **characterized in that** the inactivation of allergens is the blocking of mite or pollen allergen.

6. The polysaccharide derivative for use according to any one of claims 1 to 5 **characterized in that** the polysaccharide derivative is an aerosol.

7. The polysaccharide derivative for use according to any one of claims 1 to 3, **characterized in that** the polysaccharide derivative is contained in a mask.

8. The polysaccharide derivative for use according to any one of claims 1 to 3, **characterized in that** the polysaccharide derivative is contained in a sheet.

9. The polysaccharide derivative for use according to any one of claims 1 to 3, **characterized in that** the polysaccharide derivative is contained in a cosmetic product.

10. The polysaccharide derivative for use according to claim 1, **characterized in that** allergen-existing environment is treated with the polysaccharide derivative.

## Patentansprüche

1. Polysaccharid-Derivat mit einem Celluloseether oder einem Stärkeether als dessen Rückgrat, wobei einige oder alle Wasserstoffatome in den Hydroxygruppen des Polysaccharid-Derivats durch eine Gruppe mit der folgenden allgemeinen Formel (1) substituiert sind:

$$-E^1-(OA)_n-E^2-R \qquad (1)$$

worin $E^1$ für eine Alkylengruppe mit 1 bis 6 Kohlenstoffatomen, optional Hydroxygruppen- oder Oxogruppen-substituiert, steht; n für eine Zahl von 0 bis 50 steht; A unabhängig voneinander für Alkylen mit 1 bis 6 Kohlenstoffatomen steht, wobei die Anzahl von A = n ist; $E^2$ für eine Etherbindung oder eine Oxycarbonylgruppe steht; R für eine Alkylgruppe mit 4 bis 30 Kohlenstoffatomen, optional Hydroxygruppen-substituiert, eine Sulfoalkylgruppe mit 1 bis 5 Kohlenstoffatomen, optional Hydroxygruppen-substituiert, steht,
oder ein Salz davon zur Verwendung bei der Inaktivierung von Allergenen.

2. Polysaccharid-Derivat zur Verwendung gemäß Anspruch 1, **dadurch gekennzeichnet, dass** das zu inaktivierende

Allergen aus Pflanzenallergen, Tierallergen, Pilzen, Bakterium und Hausstaub ausgewählt ist.

3. Polysaccharid-Derivat zur Verwendung gemäß Anspruch 1 oder Anspruch 2, **dadurch gekennzeichnet, dass** der Celluloseether oder Stärkeether Hydroxyethylcellulose ist.

4. Polysaccharid-Derivat zur Verwendung gemäß mindestens einem der Ansprüche 1 bis 3, **dadurch gekennzeichnet, dass** die Inaktivierung der Allergene eine Detoxikation von einem Milben- oder einem Pollenallergen ist.

5. Polysaccharid-Derivat zur Verwendung gemäß mindestens einem der Ansprüche 1 bis 3, **dadurch gekennzeichnet, dass** die Inaktivierung der Allergene das Blockieren von einem Milben- oder einem Pollenallergen ist.

6. Polysaccharid-Derivat zur Verwendung gemäß mindestens einem der Ansprüche 1 bis 5, **dadurch gekennzeichnet, dass** das Polysaccharid-Derivat ein Aerosol ist.

7. Polysaccharid-Derivat zur Verwendung gemäß mindestens einem der Ansprüche 1 bis 3, **dadurch gekennzeichnet, dass** das Polysaccharid-Derivat in einer Maske enthalten ist.

8. Polysaccharid-Derivat zur Verwendung gemäß mindestens einem der Ansprüche 1 bis 3, **dadurch gekennzeichnet, dass** das Polysaccharid-Derivat in einer Folie (einem Sheet) enthalten ist.

9. Polysaccharid-Derivat zur Verwendung gemäß mindestens einem der Ansprüche 1 bis 3, **dadurch gekennzeichnet, dass** das Polysaccharid-Derivat in einem kosmetischen Produkt enthalten ist.

10. Polysaccharid-Derivat zur Verwendung gemäß Anspruch 1, **dadurch gekennzeichnet, dass** eine Umgebung mit vorhandenen Allergenen mit dem Polysaccharid-Derivat behandelt wird.

**Revendications**

1. Dérivé de polysaccharide ayant un éther de cellulose ou un éther d'amidon comme squelette, dans lequel une partie ou la totalité des atomes d'hydrogène dans le groupe hydroxy du dérivé de polysaccharide sont substitués par un groupe représenté par la formule générale (1) suivante :

$$-E^1- (OA)_n-E^2-R \qquad (1)$$

dans laquelle $E^1$ représente un alkylène contenant 1 à 6 atomes de carbone éventuellement substitué par un groupe hydroxy ou un groupe oxo ; n représente un nombre de 0 à 50 ; A représente indépendamment un alkylène contenant 1 à 6 atomes de carbone, le nombre de A étant n ; $E^2$ représente une liaison éther ou un groupe oxycarbonyle ; R représente un groupe alkyle contenant 4 à 30 atomes de carbone éventuellement substitué par un groupe hydroxy, un groupe sulfoalkyle contenant 1 à 5 atomes de carbone éventuellement substitué par un groupe hydroxy, ou un sel de celui-ci destiné à une utilisation dans l'inactivation des allergènes.

2. Dérivé de polysaccharide destiné à une utilisation selon la revendication 1, **caractérisé en ce que** l'allergène à inactiver est choisi parmi un allergène végétal, un allergène animal, les champignons, une bactérie, et la poussière de maison.

3. Dérivé de polysaccharide destiné à une utilisation selon la revendication 1 ou la revendication 2, **caractérisé en ce que** ledit éther de cellulose ou éther d'amidon est de l'hydroxyéthylcellulose.

4. Dérivé de polysaccharide destiné à une utilisation selon l'une quelconque des revendications 1 à 3, **caractérisé en ce que** l'inactivation des allergènes est la détoxication d'un allergène d'acarien ou de pollen.

5. Dérivé de polysaccharide destiné à une utilisation selon l'une quelconque des revendications 1 à 3, **caractérisé en ce que** l'inactivation des allergènes est le blocage d'un allergène d'acarien ou de pollen.

6. Dérivé de polysaccharide destiné à une utilisation selon l'une quelconque des revendications 1 à 5, **caractérisé en ce que** le dérivé de polysaccharide est un aérosol.

7. Dérivé de polysaccharide destiné à une utilisation selon l'une quelconque des revendications 1 à 3, **caractérisé en ce que** le dérivé de polysaccharide est contenu dans un masque.

8. Dérivé de polysaccharide destiné à une utilisation selon l'une quelconque des revendications 1 à 3, **caractérisé en ce que** le dérivé de polysaccharide est contenu dans une feuille.

9. Dérivé de polysaccharide destiné à une utilisation selon l'une quelconque des revendications 1 à 3, **caractérisé en ce que** le dérivé de polysaccharide est contenu dans un produit cosmétique.

10. Dérivé de polysaccharide destiné à une utilisation selon la revendication 1, **caractérisé en ce que** l'environnement de l'allergène existant est traité avec le dérivé de polysaccharide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1191039 A
- WO 9110434 A
- JP 58135805 A
- WO 0073351 A
- JP 6279273 A
- JP 11292714 A